(19) Europäisches Patentamt
European Patent Office
Office européen des brevets

(11) **EP 3 871 719 B1**

(12) **EUROPEAN PATENT SPECIFICATION**

(45) Date of publication and mention
of the grant of the patent:
**27.09.2023 Bulletin 2023/39**

(21) Application number: **19875609.0**

(22) Date of filing: **24.10.2019**

(51) International Patent Classification (IPC):
**A61M 16/00** (2006.01)  **A61M 16/16** (2006.01)
**A61M 16/06** (2006.01)  **A61M 16/08** (2006.01)

(52) Cooperative Patent Classification (CPC):
**A61M 16/0066; A61M 16/024; A61M 16/16;**
A61M 16/0666; A61M 16/0875; A61M 2016/003;
A61M 2205/3331; A61M 2205/3368; A61M 2205/50

(86) International application number:
**PCT/CN2019/113126**

(87) International publication number:
**WO 2020/083351 (30.04.2020 Gazette 2020/18)**

(54) **VENTILATION THERAPY APPARATUS**

ATEMTHERAPIEVORRICHTUNG

APPAREIL DE THÉRAPIE PAR VENTILATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **26.10.2018 CN 201811261603**

(43) Date of publication of application:
**01.09.2021 Bulletin 2021/35**

(73) Proprietor: **BMC Medical Co., Ltd.
Shijingshan
Beijing 100041 (CN)**

(72) Inventors:
- **WANG, Qingsong
  Beijing 100041 (CN)**
- **ZHUANG, Zhi
  Beijing 100041 (CN)**

(74) Representative: **Grünecker Patent- und
Rechtsanwälte
PartG mbB
Leopoldstraße 4
80802 München (DE)**

(56) References cited:
WO-A1-93/09834       WO-A1-2009/146484
WO-A1-2011/141845    WO-A2-02/20076

EP 3 871 719 B1

**Description**

CROSS REFERENCE TO RELEVANT APPLICATIONS

The present disclosure claims the priority of the Chinese patent application filed by State Intellectual Property Office of The P.R.C on October 26th, 2018 with the application number of 201811261603.1, and the title of " VENTILATION THERAPYAPPARATUS AND CONTROL METHOD'.

TECHNICAL FIELD

The present disclosure relates to the medical equipment field and, more particularly, to a ventilation therapy apparatus and a control method.

BACKGROUND

In modern clinical medicine, ventilation therapy apparatuses play a very important role in the field of modern medicine. Examples of ventilation therapy apparatus are shown in WO 2009/146484 A1, WO 93709834 A1, WO 02720076 A1 and WO 2011/141845 A1. Ventilation therapy apparatus is a vital medical apparatus that can prevent and treat respiratory failure, reduce complications, and save and prolong the lives of patients, which may mix pure oxygen with air and provide to the patients.

The therapy mode of the conventional ventilation therapy apparatus is to form an enclosed gas path between the patient and the ventilation therapy apparatus, namely to form a sealed state with the patient's facial area through a patient interface (usually a ventilation mask), the gas exhaled by the patient can be discharged by a specific gas path or a specially designed vent, therefore when the ventilation mask and a respiratory pipe are well worn, an exhaust channel of the patient is fixed. The ventilation therapy apparatus with this therapy mode may directly control an output pressure of the ventilation therapy apparatus through monitoring a pressure at the patient, and keep the pressure at the patient end to be equal to an expected output pressure value.

However, if the ventilation therapy apparatus constructs a semi-open gas path, that is, the patient interface and the patient are not sealed, the gas outputted by the ventilation therapy apparatus may directly leak through a gap between the patient interface and the patient's nasal cavity. At this time, the ventilation therapy apparatus is unable to directly monitor or obtain the pressure at the end of the gas path, and therefore it cannot control the pressure at the end of the gas path.

SUMMARY

The present disclosure provides a ventilation therapy apparatus, to solve the problem that in the semi-open gas path, the ventilation therapy apparatus in the prior art cannot monitor the pressure at the end of the gas path, and thus cannot achieve the control of the pressure at the end of the gas path.

In order to solve the above technical problem, the present disclosure is realized as follows:

A ventilation therapy apparatus according to the invention is defined in claim 1.

In the embodiment of the present invention, it is capable to determine the actual pressure value at the patient interface by the output pressure value and the output flow value of the signal collection point of the apparatus body, and adjust the output flow of the apparatus body according to comparison between the actual pressure value and the target pressure value, therefore the gas pressure of the airflow received by the patient may reach a preset target pressure range, and achieve the therapeutic effect.

The first calculation module comprises:

a gas resistance pressure acquisition module, configured for acquiring a gas resistance pressure value from the signal collection point to the patient interface; and
a second calculation module, configured for subtracting the gas resistance pressure value from the output pressure value, and obtaining the actual pressure value.

In the embodiment of the present disclosure, it may obtain accurate actual pressure value by acquiring the gas resistance pressure value from the signal collection point to the patient interface, and subtracting the gas resistance pressure value from the output pressure value.

The gas resistance pressure acquisition module comprises:

a gas resistance characteristic acquisition module, configured for, acquiring, under different pressure states, corresponding test flow values through a flow acquisition module when the patient interface is vacant, and acquiring a gas resistance characteristic from the signal collection point to the patient interface, wherein the gas resistance characteristic includes a correspondence relationship between the output pressure value and the output flow value; a gas resistance pressure acquisition unit configured for, acquiring the corresponding gas resistance pressure value, according to the output flow value of the apparatus body in working state and the corresponding gas resistance characteristic;

wherein the second calculation module is further configured for, subtracting the corresponding gas resistance pressure value from the output pressure value of the apparatus body, and obtaining the actual pressure value.

In the embodiment of the present disclosure, the flow acquisition module may accurately acquire, under different pressure states, the correspondence relationship between the output pressure value and the output flow value from the signal collection point to the patient interface when the patient interface is vacant. The gas resistance pressure value obtained thereby is more accurate, which in turn makes the actual pressure value more accurate.

Optionally, the signal collection point is disposed at the gas outlet of the apparatus body.

In the embodiment of the present disclosure, the flow acquisition module may acquire the gas resistance characteristic from the signal collection point to the patient interface, the signal collection point is disposed at the gas outlet of the apparatus body, which may more accurately obtain an actual input pressure or flow at the first end of the respiratory pipe.

Optionally, the target pressure value comprises a target pressure value of an inspiratory phase and a target pressure value of an exhalation phase;

the ventilation therapy apparatus includes a determination module, the determination module is configured for determining a respiratory phase according to the output pressure value and the output flow value acquired by the signal acquisition module, the respiratory phase includes the inspiratory phase and the exhalation phase;
when the determination module determines that the current is the inspiratory phase, the first control module adjusts the output flow of the apparatus body according to the actual pressure value and the target pressure value of the inspiratory phase; and
when the determination module determines that the current is the exhalation phase, the first control module adjusts the output flow of the apparatus body according to the actual pressure value and the target pressure value of the exhalation phase.

In the embodiment of the present disclosure, when the patient is inhaling, the total flow outputted by the ventilation therapy apparatus is larger than the total flow by the ventilation therapy apparatus when the patient is exhaling. If the actual pressure value is less than the target pressure value, then it is determined that the patient is inhaling. Adjusting the output flow of the apparatus body according to the respiratory requirements corresponding to the inspiratory phase and the exhalation phase in the respiratory phase, respectively, therefore it may be supplied on demand, and avoid waste.

Optionally, the apparatus body further comprises a positive pressure gas source and a humidifier, the positive pressure gas source is configured for providing an output gas, and the humidifier is configured for heating and humidifying the output gas, wherein the humidifier is connected to an output end of the positive pressure gas source.

In the embodiment of the present application, the humidifier is used to heat and humidify the gas provided by the positive pressure gas source, therefore it may meet the respiratory requirements of the user and improve the respiratory effect.

Optionally, the positive pressure gas source comprises a gas source body capable of outputting gas with a preset flow, and/or a centrifugal fan configured for pressurize air, wherein the maximum rotation speed of the centrifugal fan is larger than or equal to 20000r/min.

In the embodiment of the present disclosure, the positive pressure gas source includes the gas source body capable of outputting gas with the preset flow, and/or the centrifugal fan configured for pressurize air, the method of obtaining the positive pressure gas source is simply and convenient.

Optionally, the respiratory pipe further comprises a heating element configured for heating gas passing through the respiratory pipe, the rated power of the heating element is larger than 20 watts.

In the embodiment of the present disclosure, the heating element configured for heating gas passing through the respiratory pipe is disposed in the respiratory pipe, which may heat the temperature of the output gas in a cold environment, and improve the respiratory experience of the patient.

Optionally, the respiratory pipe further comprises a temperature sensor, configured for monitoring the temperature of the gas passing through the respiratory pipe.

In the embodiment of the present disclosure, the temperature sensor may monitor the temperature of the gas passing through the respiratory pipe in real time, therefore the ventilation therapy apparatus according to the monitored temperature, carries out the operation of correspondingly controlling the heating element to heat gas, and stops heating at the same time when the temperature is too high, which may improve the respiratory experience of the patient.

Optionally, the respiratory pipe and the apparatus body are connected through a gas path and a circuit, and the circuit and the gas path are on and off simultaneously.

In the embodiment of the present disclosure, the respiratory pipe and the apparatus body are connected through the gas path, which may output the gas provided by the apparatus body to the patient. In addition, the respiratory pipe and the apparatus body are connected through the circuit, and the electrical device in the respiratory pipe may also be electrically connected to the apparatus body, to realize the corresponding functions of the electrical device.

Optionally, the ventilation therapy apparatus comprises a second control module;

> when the patient interface is worn on the patient's nasal cavity, the second control module is configured for adjusting the output pressure value of the apparatus body to the target pressure value; and
> when the patient interface is not worn on the patient's nasal cavity, the second control module is configured for adjusting the output pressure value of the apparatus body to a preset pressure value, or the second control module is configured for controlling the apparatus body to stop running.

In the embodiment of the present disclosure, when the patient interface is worn on the patient's nasal cavity, the second control module is configured for automatically adjusting the output pressure value of the apparatus body to the target pressure value, therefore the patient may accept oxygen supply quickly. When the patient did not use an oxygen supply system of the ventilation therapy apparatus, the patient interface is exposed to the air, meanwhile, the ventilation therapy apparatus may continue to output a smaller output flow, to ensure consistent temperature and humidity inside the respiratory pipe, or directly control the apparatus body to stop running, which may save power.

It is disclosed a method for controlling the ventilation therapy apparatus, which is not part of the invention

In the example of the present disclosure, it is capable to determine the actual pressure value at the patient interface by the output pressure value and the output flow value of the signal collection point of the apparatus body, and adjust the output flow of the apparatus body according to comparison between the actual pressure value and the target pressure value, therefore the gas pressure of the airflow received by the patient may reach a preset target pressure range, and achieve the therapeutic effect.

The step of calculating an actual pressure value at the patient interface according to the output pressure value and the output flow value of the signal collection point, comprises:

> acquiring a gas resistance pressure value from the signal collection point to the patient interface; and
> subtracting the gas resistance pressure value from the output pressure value, and obtaining the actual pressure value.

In the example of the present disclosure, it may obtain accurate actual pressure value by acquiring the gas resistance pressure value from the signal collection point to the patient interface, and subtracting the gas resistance pressure value from the output pressure value.

The step of calculating an actual pressure value at the patient interface according to the output pressure value and the output flow value of the signal collection point, comprises:

> acquiring the output pressure value and the output flow value of the signal collection point of an apparatus body;
> acquiring, under different pressure states, corresponding test flow values when the patient interface is vacant, and acquiring a gas resistance characteristic from the signal collection point to the patient interface, wherein the gas resistance characteristic includes a correspondence relationship between the output pressure value and the output flow value;
> acquiring the corresponding gas resistance pressure value according to the output flow value of the apparatus body in working state and the corresponding gas resistance characteristic; and
> subtracting the corresponding gas resistance pressure value from the output pressure value of the apparatus body, and obtaining the actual pressure value.

In the example of the present disclosure, the gas resistance pressure acquisition module may accurately acquire, under different pressure states, the correspondence relationship between the gas resistance pressure value and the output flow value from the signal collection point to the patient interface when the patient interface is vacant. The gas resistance pressure value obtained thereby is more accurate, which in turn makes the actual pressure value more accurate.

Optionally, the method further comprises:

> when the patient interface is worn on the patient's nasal cavity, adjusting the output pressure value of the apparatus body to the target pressure value; and

when the patient interface is not worn on the patient's nasal cavity, adjusting the output pressure value of the apparatus body to a preset pressure value which is less than the target pressure value, or controlling the apparatus body to stop running.

In the example of the present disclosure, when the patient interface is worn on the patient's nasal cavity, the second control module is configured for automatically adjusting the output pressure value of the apparatus body to the target pressure value, therefore the patient may accept oxygen supply quickly. When the patient did not use an oxygen supply system of the ventilation therapy apparatus, the patient interface is exposed to the air, meanwhile, the ventilation therapy apparatus may continue to output a smaller output flow, to ensure consistent temperature and humidity inside the respiratory pipe, or directly control the apparatus body to stop running, which may save power.

As an example, not being part of the invention, after acquiring an output pressure value and an output flow value of a signal collection point of an apparatus body, the method further comprises:

determining a respiratory phase according to the output pressure value and the output flow value, wherein the respiratory phase includes the inspiratory phase and the exhalation phase;
acquiring a target pressure value of the inspiratory phase and a target pressure value of the exhalation phase at the patient interface;
if it is determined that the he current is inspiratory phase, adjusting the output flow of the apparatus body according to the actual pressure value and the target pressure value of the inspiratory phase; and
if it is determined that the he current is exhalation phase, adjusting the output flow of the apparatus body according to the actual pressure value and the target pressure value of the exhalation phase.

In the example of the present disclosure, when the patient is inhaling, the total flow outputted by the ventilation therapy apparatus is larger than the total flow by the ventilation therapy apparatus when the patient is exhaling. If the actual pressure value is less than the target pressure value, then it is determined that the patient is inhaling. According to the respiratory requirements respectively corresponding to the inspiratory phase and the exhalation phase in the respiratory phase, adjusting the output flow of the apparatus body respectively, therefore it may be supplied on demand, and avoid waste.

Further, a computer program is disclosed, not being part of the invention, comprising a computer readable code, when the computer readable code is run on a computing processing device, causing the computing processing device to execute the method for controlling the ventilation therapy apparatus anyone of the above ventilation therapy apparatus.

A computer readable medium is also disclosed, storing the above computer program.

The ventilation therapy apparatus provided in the embodiment of the present disclosure, includes an apparatus body, a respiratory pipe and a patient interface. The apparatus body further includes a signal acquisition module, a target pressure acquisition module and a first control module. The signal acquisition module is configured for acquiring the output pressure value and the output flow value of the signal collection point of the apparatus body; the target pressure acquisition module is configured for acquiring the target pressure value at the patient interface; the first calculation module is configured for calculating an actual pressure value at the patient interface according to the output pressure value and the output flow value of the signal collection point; the first control module is configured for adjusting the output flow of the apparatus body according to the actual pressure value and the target pressure value; when the actual pressure value is larger than the target pressure value, the first control module may reduce the output flow of the apparatus body; and when the actual pressure value is less than the target pressure value, the first control module may rise the output flow of the apparatus body. In the present disclosure, it is capable to determine the actual pressure value at the patient interface by the output pressure value and the output flow value of the signal collection point of the apparatus body, and adjust the output flow of the apparatus body according to comparison between the actual pressure value and the target pressure value, therefore the gas pressure of the airflow received by the patient may reach a preset target pressure range, and achieve the therapeutic effect.

The above description is only an overview of the technical solution of the present disclosure, in order to understand the technical means of the present disclosure more clearly, it may be implemented in accordance with the content of the specification, and in order to make the above and other purposes, features and advantages of the present disclosure more obvious and understandable, the following specifically cites the specific implementation of the present disclosure.

BRIEF DESCRIPTION OF THE DRAWINGS

In order to more clearly explain the technical solutions in the embodiments of the present disclosure or the prior art, the following will briefly introduce the drawings that need to be used in the description of the embodiments or the prior art. Obviously, the drawings in the following description are some embodiments of the present disclosure. For those of ordinary skill in the art, other drawings may be obtained based on these drawings without creative work.

Fig. 1 is a structural block diagram of the ventilation therapy apparatus according to an embodiment of the present disclosure;

Fig. 2 is a schematic diagram showing a flow-time of the patient's respiratory process according to an embodiment of the present disclosure;

Fig. 3 is a structural block diagram of the first calculation module according to an embodiment of the present disclosure;

Fig. 4 is a schematic diagram showing a flow-pressure drop of the patient's respiratory process according to an embodiment of the present disclosure;

Fig. 5 is a flow chart showing steps of the method for controlling the ventilation therapy apparatus according to the present disclosure;

Fig. 6 is a computing processing device that may implement the method according to the present disclosure; and

Fig. 7 is a portable or fixed storage module according to an example of the present disclosure.

DETAILED DESCRIPTION

In order to make the purpose, technical solutions and advantages of the embodiments of the present disclosure clearer, the technical solutions in the embodiments of the present disclosure will be described clearly and completely in conjunction with the accompanying drawings in the embodiments of the present disclosure.

The following describes in detail the ventilation therapy apparatus and the control method according to the present disclosure by listing several specific embodiments.

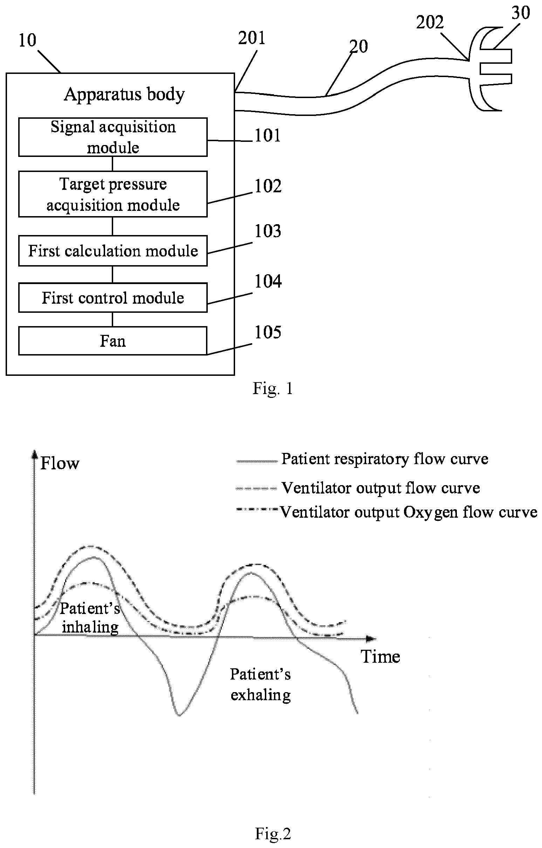

Referring to Fig. 1, there is shown a structural block diagram of the ventilation therapy apparatus according to an embodiment of the present disclosure, comprising an apparatus body 10, configured for outputting gas with a preset pressure and a preset flow, and the apparatus body 10 includes a gas outlet; a respiratory pipe 20, the respiratory pipe 20 including a first end 201 and a second end 202 which communicates with each other, wherein the first end 201 of the respiratory pipe 20 communicates with the gas outlet; a patient interface 30, wherein the second end 202 of the respiratory pipe 20 is connected to the patient interface 30, the patient interface 30 is configured for being worn on a patient's nasal cavity, when the patient interface 30 is worn on the patient's nasal cavity, a gas outlet gap is disposed between the patient interface 30 and the patient's nasal cavity.

Wherein, the ventilation therapy apparatus may be applied in an open gas path, and also may be applied in a semi-open gas path. The semi-open gas path thereof includes a situation that the patient interface 30 is worn on the patient's nasal cavity, a vacant state of the patient interface 30 resulting by that the patient interface 30 is not worn on the patient's nasal cavity, which is a complete open gas path. In the embodiment of the present disclosure, it may adjust correspondingly the output flow of the apparatus body 10 by the comparison result of an actual pressure value and a target pressure value at the patient interface 30.

In addition, Referring to Fig. 1, the structural of the patient interface 30 includes two branch tubes divided at the end, which deliver gas to the two nostrils of the patient separately. An inner diameter of the branch tube at the end of the patient interface 30 is larger than 4 mm, a length is larger than 4 mm, and a thinnest part of a tube wall is less than 0.5 mm. Because these two branch tubes are not an oxygen suction tubes, the flow rate of the oxygen suction tube is usually only 5 to 15 liters per minute, however, these two branch tubes need a large enough airflow (more than 60 liters per minute (LPM)) to generate the required positive pressure, therefore their output flow is relatively large, so the inner diameter is larger than an inner diameter of the conventional oxygen suction tube. But if an outer diameter of the branch tube at the end is also thick, it will hinder the nostril exhalation, and contact with an inner wall will make the patient feel uncomfortable. Therefore, the tube wall should be as thin as possible and not take up an exhaust area of the nostril.

Wherein, the apparatus body 10 further comprises: a signal acquisition module 101, configured for acquiring an output pressure value and an output flow value of a signal collection point of the apparatus body 10.

Specifically, the second end 202 of the respiratory pipe 20 is configured for outputting the gas provided by the apparatus body 10, and the pressure and the flow of the gas outputted may be controlled. A fan 105 may also be disposed inside the apparatus body 10, the fan 105 may be driven by a motor to rotate at a corresponding preset rotating speed, therefore the external air is sucked in the apparatus body 10, and the airflow with different pressure and flow thresholds is outputted through the respiratory pipe 20.

Preferably, the signal acquisition module 101 is configured for acquiring the output pressure value and the output flow value of the signal collection point of the apparatus body 10. Wherein, the signal collection point of the apparatus body 10 may be the gas outlet of the apparatus body 10 or a position close to the gas outlet, and preferably, is configured to obtain the actual input pressure $P_1$ or flow rate $F_0$ of the first end 201 of the respiratory pipe 20.

A target pressure acquisition module 102, acquiring a target pressure value at the patient interface 30. The target pressure value may be a pressure value inputted directly into the ventilation therapy apparatus according to the patient's respiratory state, may also be a pressure value suitable for the patient's state automatically learned by the ventilation therapy device based on the patient's respiratory state for a period of time. The target pressure value may be a specific value or a threshold value suitable for the patient's state.

It should be noted that the target pressure value is larger than 0, that is, the target pressure value is larger than the atmospheric pressure value. The target pressure value is larger than the atmospheric pressure value because the ventilation therapy apparatus is applied in the open gas path or the semi-open gas path, and positive pressure must be maintained in the nasal cavity to ensure that the human body does not directly inhale external air.

In practical applications, referring to Fig. 2, there is shown a schematic diagram showing a flow-time of the patient's respiratory process according to the present disclosure. When the patient uses the ventilation therapy apparatus to breathe, the total flow of the inhaled gas will change with time. Therefore, for a conventional ventilation therapy apparatus, in order to optimize the patient's respiratory experience, the patient may be provided with two different levels positive pressure during the patient's exhalation and inhalation. For a high-flow oxygen therapy apparatus, it may provide a larger flow when the patient inhales, to facilitate the patient inhale more gas, and provide a smaller flow when exhaling, to avoid blockage of the patient's airway. At the same time, for the high-flow oxygen therapy apparatus, oxygen supply is introduced. Therefore, the high-flow oxygen therapy apparatus may also ensure a constant oxygen concentration in the outputted gas to ensure a stable therapeutic effect.

In the embodiment of the present disclosure, before the patient uses the ventilation therapy apparatus, a target pressure value $P_t$ will be preset according to their own conditions, and the target pressure value $P_t$ is the pressure at the patient interface in an ideal state. According to the airflow pressure $P_1$ and the airflow flow $F_0$ outputted by the apparatus body 10, the actual pressure $P_2$ at the patient interface may be calculated. Through real-time monitoring of $P_2$ and comparing $P_2$ with the target pressure value $P_t$, it may be determined which preset state the apparatus body is in, that is the exhalation state or the inhalation state, and according to the current state of the apparatus body, the fan of the apparatus body 10 is controlled to run at the corresponding preset rotating speed, and outputs the airflow corresponding to the preset threshold, for example, when it is determined that the patient is inhaling, the air supply system of the ventilation therapy apparatus outputs a flow that is slightly larger than the patient's inhalation volume for auxiliary inhalation; when the patient is determined to exhale, the air supply system of the ventilation therapy apparatus outputs a smaller flow rate, to prevent the patient's exhaled gas from flowing back to the ventilation therapy apparatus.

It should be noted that the target pressure value $P_t$ needs to be a positive pressure value, that is, the target pressure value $P_t$ is larger than the atmospheric pressure value. Because in the embodiment of the present disclosure, the ventilation therapy apparatus uses an open gas path, it is necessary to ensure that the positive pressure is maintained in the nasal cavity, to ensure that the human body will not directly inhale outside air.

A first calculation module 103, calculating an actual pressure value at the patient interface according to the output pressure value and the output flow value of the signal collection point.

In the embodiment of the present disclosure, the air flow characteristics in the respiratory pipe 20 are certain, according to an energy equation of a fluid, a steady flow of the incompressible fluid in the tube has the following formula:

$$\frac{U^2}{2} + \frac{p}{\rho} + e + \Pi = const$$

Wherein, U is a flow rate of the fluid, p is a pressure of the fluid, $\rho$ is a density of the fluid, e is an internal energy of the fluid, $\Pi$ is a potential energy, and const is a constant, which means that in a fluid system, such as airflow and water flow, the faster the flow rate, the less the pressure generated by the fluid.

In addition, an air flow resistance has the following formula:

$$F = \frac{1}{2} C \rho S^{\,2}$$

Wherein, F is an air resistance, $\rho$ is the density of the fluid, C is a resistance coefficient, S is a windward area.

Therefore, combining the inferences derived from the above two formulas, after an incompressible fluid flows through a tube with a length of L and a lateral area of S at a certain flow rate, the pressure changes as follows:

$$\Delta P = P_1 - P_2 = \rho(e_2 - e_1) = \rho \times F \times L = \frac{1}{2}C\rho^2 S U^2 L \propto U^2$$

That is, the pressure drop of the incompressible fluid (the pressure drop is a value of the pressure $P_1$ at the first end 201 of the respiratory pipe 20 minus the pressure at the patient interface) and the flow rate are quadratic. However, the gas is a compressible fluid, so when the pressure drops, the density $\rho$ will increase slightly.

Therefore, either the theoretical output flow rate $F_t$ may be calculated from $P_1$, or the pressure drop $\Delta P$ of the air flow through the tube may be calculated from $F_0$, and according to the formula $P_1 = \Delta P + P_2$, the value of the airflow pressure $P_2$ at the patient interface may be obtained.

A first control module 104, adjusting an output flow of the apparatus body 10 according to the actual pressure value and the target pressure value; when the actual pressure value is larger than the target pressure value, reducing the output flow of the apparatus body 10; and when the actual pressure value is less than the target pressure value, rising the output flow of the apparatus body 10.

Specifically, in the embodiment of the present disclosure, before the patient uses the air supply system of the ventilation therapy apparatus, the target pressure value $P_t$ will be preset according to their own conditions, and the target pressure value $P_t$ is the pressure value in an ideal state free from the interference of the pressure drop. When the patient uses the ventilation therapy apparatus, due to the interference of the pressure drop, which will cause the actual pressure $P_2$ at the patient interface is different from the target pressure value $P_t$, and the influence of this pressure drop on the actual pressure $P_2$ at the patient interface may be determined by the first control module 104 based on the comparison result between $P_2$ and $P_t$. If the first control module 104 determines that the actual pressure $P_2$ at the patient interface is larger than the target pressure value $P_t$, it is determined that the current working state of the apparatus body 10 is the exhalation state, meanwhile a control instruction is sent to the motor by the first control module 104, therefore the motor drives the fan 105 to work at a lower rotating speed, and output a smaller first output threshold airflow, to prevent the patient's exhaled gas from flowing back to the ventilation therapy apparatus, until the actual pressure value is equal to the target pressure value.

It should be noted that, the first control module 104 may also be a compensation module independent of the apparatus body 10, to output a compensation airflow to the respiratory pipe 20. For example, the first control module 104 may be a compensation fan or a compensation gas cylinder disposed independently of the fan 105, when the actual pressure value $P_2$ is larger than the target pressure value $P_t$, outputs the corresponding compensation air flow, therefore the apparatus body 10 outputs the air flow at a smaller first output threshold; when the actual pressure value $P_2$ is less than the target pressure value $P_t$, outputs the corresponding compensation air flow, therefore the apparatus body 10 outputs the air flow at a larger second output threshold.

Specifically, if the first control module 104 determines that the actual pressure $P_2$ at the patient interface is less than the target pressure value $P_t$, then it is determined that the current working state of the apparatus body 10 is the inhalation state. At this time, a control instruction is sent to the motor by the first control module 104, therefore the motor drives the fan 105 to work at a relatively high rotating speed, and output a larger second output threshold airflow, to perform auxiliary inhalation.

In summary, a ventilation therapy apparatus according to the embodiment of the present disclosure, includes: an apparatus body, a respiratory pipe and a patient interface. The apparatus body further includes: a signal acquisition module, a target pressure acquisition module and a first control module. The signal acquisition module is configured for acquiring the output pressure value and the output flow value of the signal collection point of the apparatus body; the target pressure acquisition module is configured for acquiring the target pressure value at the patient interface; the first calculation module is configured for, calculating the actual pressure value at the patient interface according to the output pressure value and the output flow value of the signal collection point; the first control module is configured for adjusting an output flow of the apparatus body according to the actual pressure value and the target pressure value. In the present disclosure, it is capable to determine the actual pressure value at the patient interface by the output parameters feedback of the signal collection point of the apparatus body, determine the patient's respiratory state according to the comparison between the actual pressure value and the target pressure value, and output the gas with a corresponding threshold, therefore the gas pressure of the airflow received by the patient may reach a preset target pressure range, and achieve the therapeutic effect.

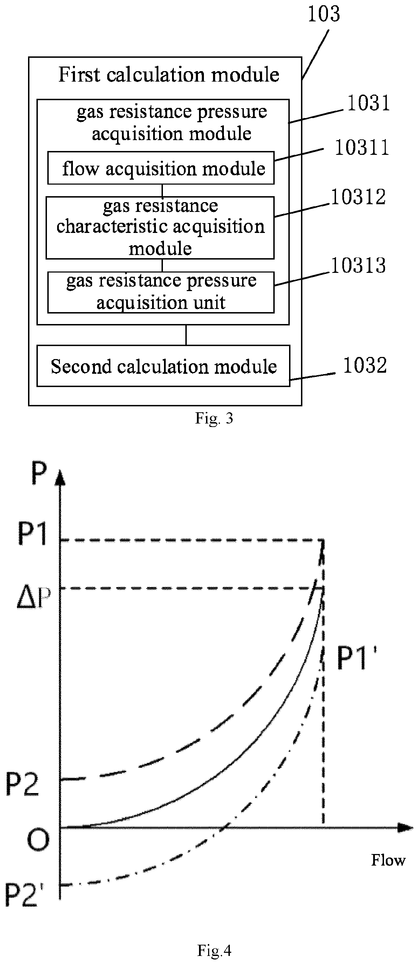

Referring to Fig. 3, it is shown a structural block diagram of the first calculation module according to an embodiment of the present disclosure, the first calculation module 103 comprises:

a gas resistance pressure acquisition module 1031, configured for acquiring a gas resistance pressure value from the signal collection point to the patient interface. The gas resistance pressure acquisition module 1031 is configured for acquiring the pressure value and the flow value of the signal collection point in the apparatus body 10, wherein the signal collection point in the apparatus body 10 may be the gas outlet of the apparatus body 10 or a position close to the gas outlet, and preferably, is configured to obtain the actual input pressure $P_1$ or flow rate $F_0$ of the first end 201 of the respiratory pipe 20.

A second calculation module 1032, configured for subtracting the gas resistance pressure value from the output pressure value, and obtaining the actual pressure value. Specifically, according to the above formula $P_1=\Delta P+P_2$, the value of the airflow pressure P2 at the patient interface may be obtained as $P_1-\Delta P$.

Referring to Fig. 3, the gas resistance pressure acquisition module 1031 comprises:

a flow acquisition module 10311 configured for, acquiring the output pressure value and the output flow value of the signal collection point of the apparatus body.

A gas resistance characteristic acquisition module 10312 configured for, acquiring, under different pressure states, corresponding test flow values through the flow acquisition module when the patient interface is vacant, and acquiring a gas resistance characteristic from the signal collection point to the patient interface, the gas resistance characteristic includes a correspondence relationship between the gas resistance pressure value and the output flow value.

A gas resistance pressure acquisition unit 10313 configured for, according to the output flow value of the apparatus body in working state and the corresponding gas resistance characteristic, acquiring the corresponding gas resistance pressure value.

The second calculation module 1032 further configured for, subtracting the corresponding gas resistance pressure value from the output pressure value of the apparatus body, and obtaining the actual pressure value.

In the embodiment of the present disclosure, when the patient is breathing, it will produce changes in airflow, therefore it will cause a pressure drop between the signal collection point and the end of the patient interface. The flow of the gas passing through the respiratory pipe 20 and the pressure drop between $\Delta P$ between the first end of the respiratory pipe and the end of the patient interface have a functional relationship $\Delta P=k*Flow^n$, wherein n is slightly less than 2. The pressures $P_1$ of the first end of the respiratory pipe and the pressure $P_2$ of the end of the patient interface have a relationship of $P_1=\Delta P+P_2$. Specifically, k and n may be constants, and the values of k and n may be measured by experiments on the circuit.

Specifically, the experiments on the circuit may include: operating the apparatus body 10, and placing the patient interface 30 in the air, at this time, the actual pressure $P_2$ at the patient interface 30 is 0. According to the formula $P_1=\Delta P+P_2$, $\Delta P=P_1$ may be obtained. Recording the flow value and the pressure drop value, multiple experiments, obtaining a correspondence relationship diagram of the flow and the pressure drop. According to the correspondence relationship diagram, the value of k and n may be obtained, that is, the correspondence relationship between the gas resistance pressure value and the output flow value is obtained. Among them, the correspondence relationship between the gas resistance pressure value and the output flow value may be a nonlinear correspondence relationship.

Preferably, when the ventilation therapy apparatus is officially working, the patient interface 30 is inserted into the patient's nasal cavity, and the air resistance pressure acquisition unit 10313 detects the working pressure value and the working flow value outputted by the apparatus body 10 itself to the first end 201 of the respiratory pipe, and according to the air resistance characteristic corresponding to the working flow value, and the corresponding air resistance pressure value is obtained. Specifically, according to the flow and the pressure drop diagram, the air resistance pressure value $\Delta P$ corresponding to the working flow value in the flow and the pressure drop diagram may be determined, and according to the formula $P_1=\Delta P+P_2$, the value of the airflow pressure $P_2$ at the patient interface may be obtained, the value of $P_2$ is the working pressure minus the air resistance pressure $\Delta P$.

Specifically, referring to Fig. 4, it is shown a schematic diagram showing a flow-pressure drop of the patient's respiratory process according to the present disclosure. When the air supply system of the ventilation therapy apparatus is officially working, the patient interface 30 is inserted into the patient's nasal cavity, and the signal acquisition module acquires the airflow pressure $P_1$ and the flow $F_0$ outputted by the ventilation therapy apparatus itself to the first end of the respiratory pipe, the flow-pressure drop diagram may be used to express the gas resistance characteristics. According to the flow-pressure drop diagram, the theoretical output flow $F_t$ may be calculated from $P_1$, or the pressure drop $\Delta P$ of the air flow through the circuit may be found from $F_0$, and according to the formula $P_1=\Delta P+P_2$, the value of the airflow pressure $P_2$ at the patient interface may be obtained.

The air resistance characteristics include an air resistance characteristic of the respiratory pipe and an air resistance characteristic of the patient interface; the air resistance characteristic of the respiratory pipe is related to the cross-sectional area of the respiratory pipe, and the air resistance characteristic of the patient interface is related to the air outlet gap. Specifically, the patient interface fixing belt is worn on the patient's face, even after being worn correctly, the gas discharge path and the area of the gas path may vary with the physiological characteristics of the patient, and may vary with the strength of the patient when wearing it.

In an experiment, the pressure and flow rate tests are carried out through three types of nasal oxygen tubes of L, M and S, the specific experimental results may be referred to Table 1, so as to understand the air resistance pressure and flow characteristics of different types of nasal oxygen tubes at work.

Table 1

| L | | M | | S | |
|---|---|---|---|---|---|
| Flow (0.1L/min) | Pressure ( pa) | Flow (0.1L/min) | Pressure ( pa) | Flow (0.1L/min) | Pressure ( pa) |
| / | / | / | / | 100 | 72 |
| 150 | 64.75 | 150 | 101.5 | 150 | 145.25 |
| 200 | 109 | 199.95 | 169.875 | 200.025 | 249 |
| 250 | 152.4 | 250.075 | 244.05 | 249.975 | 362.75 |
| 300 | 204.25 | 300 | 329.5 | 300.075 | 493.25 |

Optionally, the signal collection point is disposed at the gas outlet of the apparatus body 10. In the embodiment of the present disclosure, the signal collection point of the apparatus body 10 may be the gas outlet of the apparatus body 10 or a position close to the gas outlet, and preferably, is configured to obtain the actual input pressure $P_1$ or flow rate $F_0$ of the first end 201 of the respiratory pipe 20.

In the embodiment of the present disclosure, when the patient is inhaling, the total flow outputted by the ventilation therapy apparatus is larger than the total flow by the ventilation therapy apparatus when the patient is exhaling. If the actual pressure value is less than the target pressure value, then it is determined that the patient is inhaling, meanwhile rising the output flow of the apparatus body to assist the patient to inhale; if the actual pressure value is larger than the target pressure value, then it is determined that the patient is exhaling, and reducing the output flow of the apparatus body to prevent the patient's exhaled gas from flowing back to the ventilation therapy apparatus. If the actual pressure value is equal to the target pressure value, then maintain the current rotating speed of the fan unchanged.

In addition, in another implementation, the size relationship of the patient's respiratory flow and the output flow of the ventilation therapy apparatus may be obtained from $F=F_t-F_0$ ($F_t$ is the theoretical output flow calculated according to $P_1$, and $F_0$ is the second flow value of the signal collection point when the patient interface is inserted into the patient's nasal cavity), if $F > 0$, it means that the flow rate output by the ventilation therapy apparatus is larger than the patient's respiratory flow. At this time, the patient is in the state of exhalation, or in the state of inhalation, and the inhalation volume is all provided by the ventilation therapy apparatus; if $F < 0$, it means that the output flow of the ventilation therapy apparatus is less than the patient's inspiratory flow, and the patient will inhale some air from the environment. At this time, the patient's inhaled oxygen concentration cannot reach the disposed value. Therefore, if $F>0$ may be maintained during the operation of the ventilation therapy apparatus, and the relationship between the oxygen flow $F_{o2}$ and the total flow $F_0$ or the air flow $F_{air}$ is maintained in the relationship of the above equation, it may be ensured that the patient inhales the fixed oxygen concentration gas provided by the ventilation therapy apparatus.

In practical applications, when the patient wears the patient interface, because of its own air resistance, the patient interface will also have a certain pressure due to the patient's air resistance when not breathing. When the patient interface is removed, the patient interface is directly connected to the environment, the actual pressure $P_2$ is close to 0. Because the airway is negative pressure when inhaling, if the inhalation is strong, the pressure $P_2$ at the patient interface may drop to 0 or a negative value, but it will not be maintained for a long time, the pressure $P_2$ at the patient interface is determined to be close to 0 for a long time, it may be regarded as a non-use state.

Optionally, the ventilation therapy apparatus further comprises a second control module, when the patient interface is worn on the patient's nasal cavity, the second control module is configured for adjusting the output pressure value of the apparatus body to the target pressure value; and when the patient interface is not worn on the patient's nasal cavity, the second control module is configured for adjusting the output pressure value of the apparatus body to a preset pressure value, or the second control module is configured for controlling the apparatus body to stop running.

Therefore, in the embodiment of the present disclosure, when the pressure $P_2$ at the patient interface is close to 0, it is in the standby state, when the airflow pressure at the patient interface is close to 0 and maintained for the preset time, it means that the patient interface is not worn on the patient's nasal cavity, the patient does not use the air supply system of the ventilation therapy apparatus, and it is the standby state. When the patient interface is exposed to the air, at this time, the ventilation therapy apparatus may continue to output smaller output flow, to ensure the temperature and the humidity inside the respiratory pipe are constant, or directly control the apparatus body to stop running, to save power.

In addition, when the patient interface is worn on the patient's nasal cavity, the second control module is configured for adjusting the output pressure value of the apparatus body to the target pressure value, therefore the patient may receive oxygen supply quickly.

Optionally, the target pressure value comprises a target pressure value of an inspiratory phase and a target pressure value of an exhalation phase; the ventilation therapy apparatus includes a determination module, the determination module is configured for determining a respiratory phase according to the output pressure value and the output flow value acquired by the signal acquisition module, the respiratory phase includes the inspiratory phase and the exhalation phase; when the determination module determines that the current is the inspiratory phase, the first control module adjusts the output flow of the apparatus body according to the actual pressure value and the target pressure value of the inspiratory phase; and when the determination module determines that the current is the exhalation phase, the first control module adjusts the output flow of the apparatus body according to the actual pressure value and the target pressure value of the exhalation phase.

In the embodiment of the present disclosure, according to the schematic diagram showing the flow-time of the patient's respiratory process shown in Fig. 2, we could know that, in the process of the patient using the ventilation therapy apparatus to breathe, there are two process: an inhalation phase and an exhalation phase. The determination module is configured to determine the respiratory phase according to the output pressure value and the output flow value obtained by the signal acquisition module. Therefore, for the ordinary ventilation therapy apparatus, in order to optimize the patient's breathing experience, it may provide the patient with two different levels of positive pressure during the patient's exhalation and inhalation. For the high-flow oxygen therapy apparatus, it may provide a larger flow when the patient inhales, therefore the patient may inhale more gas, and provide a smaller flow when exhaling, so as to avoid blockage of the patient's airway.

Specifically, before the patient uses the ventilation therapy apparatus, he will preset a target pressure value $P_t$ according to his own situation, and the target pressure value $P_t$ is the pressure at the patient's interface in an ideal state. According to the airflow pressure $P_1$ and the airflow flow $F_0$ output by the apparatus body 10, the value of the actual pressure $P_2$ at the patient interface may be calculated. Through real-time monitoring of $P_2$, and comparing $P_2$ with the target pressure value $P_t$, it may be determined which preset state the apparatus body is in, that is the exhalation state or the inhalation state. When the actual pressure value $P_2$ is larger than the target pressure value $P_t$, it is determined as the exhalation phase; if the actual pressure value $P_2$ is less than the target pressure value $P_t$, it is determined as the inhalation phase.

When it is determined that the patient is inhaling, the ventilation therapy apparatus, under the control of the first control module, makes the motor drive the fan to work at a relatively large rotating speed, therefore the ventilation therapy apparatus outputs a flow that is slightly larger than the patient's inhalation volume, until the pressure value at the patient interface reaches the target pressure value of the inhalation phase, to perform auxiliary inhalation.

When it is determined that the patient is exhaling, the ventilation therapy apparatus, under the control of the first control module, makes the motor drive the fan to work at a lower rotating speed, therefore the ventilation therapy apparatus outputs a smaller flow rate, until the pressure value at the patient interface reaches the target pressure value of the exhalation stage, to prevent the patient's exhaled gas from flowing back to the ventilation therapy apparatus.

Optionally, the apparatus body further comprises a positive pressure gas source and a humidifier, the positive pressure gas source is configured for providing an output gas, and the humidifier is configured for heating and humidifying the output gas, the humidifier is connected to an output end of the positive pressure gas source.

In practical applications, the gas people breathe has a certain amount of moisture, and the breathed gas has the highest breathing comfort at a certain temperature. Therefore, the gas provided by the positive pressure gas source may be heated and humidified through the humidifier, therefore it may meet the user's breathing needs and improve the breathing effect.

Optionally, the positive pressure gas source comprises a gas source body capable of outputting gas with a preset flow, and/or a centrifugal fan configured for pressurize air, the maximum rotation speed of the centrifugal fan is larger than or equal to 20000r/min.

In the embodiment of the present disclosure, the positive pressure gas source may be a gas cylinder that stores a quantitative amount of breathing gas. In addition, the positive pressure gas source may also be outside air, and the ventilation therapy apparatus may transmit the gas provided by the positive pressure gas source through the centrifugal fan.

Optionally, the respiratory pipe further comprises a heating element configured for heating gas passing through the respiratory pipe, the rated power of the heating element is larger than 20 watts.

In the embodiment of the present disclosure, according to the different usage scenarios of the ventilation therapy apparatus, the temperature of the output gas is easily affected by the colder environment. In this case, the heating element configured to heat the gas passing through the respiratory pipe may be installed in the respiratory pipe. Preferably, the temperature of the output gas is heated in a cold environment, to improve the breathing experience of the patient.

Optionally, the respiratory pipe further comprises a temperature sensor, configured for monitoring the temperature of the gas passing through the respiratory pipe. The temperature sensor may real-time monitor the temperature of the gas in the respiratory pipe, therefore the ventilation therapy apparatus according to the monitored temperature, carries out the operation of correspondingly controlling the heating element to heat gas, and stops heating at the same time when the temperature is too high.

Optionally, the respiratory pipe and the apparatus body are connected through a gas path and a circuit, and the circuit and the gas path are on and off simultaneously.

In the embodiment of the present disclosure, the respiratory pipe and the apparatus body are connected through the gas path, which may output the gas provided by the apparatus body to the patient. In addition, the respiratory pipe and the apparatus body are connected through the circuit, and the electrical device in the respiratory pipe may also be electrically connected to the apparatus body, to realize the corresponding function of the electrical device. For example, the electrical device may include a humidifier, heating elements and temperature sensors, these devices need to be powered by the apparatus body, need to receive control signals transmitted by the apparatus body, and at the same time need to transmit corresponding status signals to the apparatus body.

In summary, the ventilation therapy apparatus according to the embodiment of the present disclosure, includes: an apparatus body, a respiratory pipe and a patient interface. The apparatus body further includes: a signal acquisition module, a target pressure acquisition module and a first control module. The signal acquisition module is configured for acquiring the output pressure value and the output flow value of the signal collection point of the apparatus body; the target pressure acquisition module is configured for acquiring the target pressure value at the patient interface; the first calculation module is configured for calculating the actual pressure value at the patient interface according to the output pressure value and the output flow value of the signal collection point; the first control module is configured for adjusting the output flow of the apparatus body according to the actual pressure value and the target pressure value. In the present disclosure, it is capable to determine the actual pressure value at the patient interface by the output parameters feedback of the signal collection point of the apparatus body, determine the patient's respiratory state according to the comparison between the actual pressure value and the target pressure value, and output the gas with a corresponding threshold, therefore the gas pressure of the airflow received by the patient may reach a preset target pressure range, achieve the therapeutic effect, and ensure the patient inhales the gas with a fixed oxygen concentration provided by the ventilation therapy apparatus. In addition, in the present disclosure, it may dispose the heating element inside the respiratory pipe, the heating element is configured for heating the gas passing through the respiratory pipe, preferably, the temperature of the output gas is heated in a cold environment, to improve the breathing experience of the patient. The gas may also be heated and humidified by the humidifier, therefore it may meet the user's breathing needs and improve the breathing effect

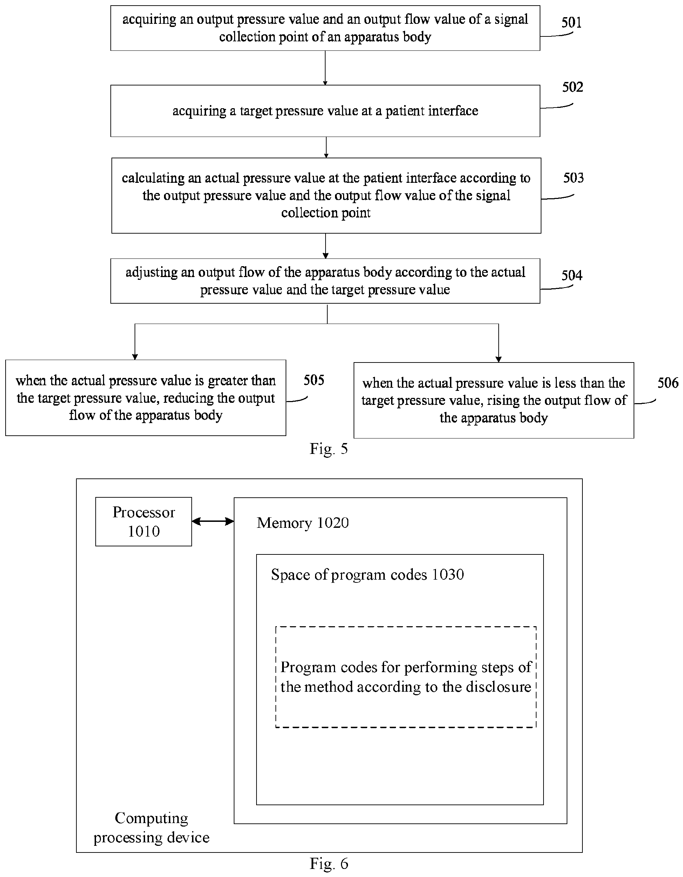

Referring to Fig. 5, it is shown a flow chart showing steps of the method for controlling the ventilation therapy apparatus according to the present disclosure, the method for controlling the ventilation therapy apparatus, the ventilation therapy apparatus constructs a semi-open gas path, wherein the method comprises:

Step 501, acquiring an output pressure value and an output flow value of a signal collection point of an apparatus body.

Specifically, in this step, the output pressure value $P_1$ and the output flow value $F_0$ of the signal collection point of the apparatus body may be obtained. Wherein, the signal collection point of the apparatus body may be the gas outlet of the apparatus body or a position close to the gas outlet, and preferably, is configured to obtain the actual input pressure $P_1$ or flow rate $F_0$ of the first end of the respiratory pipe.

Step 502, acquiring a target pressure value at a patient interface.

In the example of the present disclosure, before the patient uses the ventilation therapy apparatus, he will preset a target pressure value $P_t$ according to his own situation, and the target pressure value $P_t$ is the pressure at the patient's interface in an ideal state. According to the airflow pressure $P_1$ and the airflow flow $F_0$ output by the apparatus body, the value of the actual pressure $P_2$ at the patient interface may be calculated. Through real-time monitoring of $P_2$, and comparing $P_2$ with the target pressure value $P_t$, it may be determined which preset state the apparatus body is in, that is the exhalation state or the inhalation state, and according to the current state of the apparatus body, the fan of the apparatus body 10 is controlled to run at the corresponding preset rotating speed, and outputs the airflow corresponding to the preset threshold, for example, when it is determined that the patient is inhaling, the air supply system of the ventilation therapy apparatus outputs a flow that is slightly larger than the patient's inhalation volume for auxiliary inhalation; when the patient is determined to exhale, the air supply system of the ventilation therapy apparatus outputs a smaller flow rate, to prevent the patient's exhaled gas from flowing back to the ventilation therapy apparatus.

It should be noted that, the target pressure value $P_t$ needs to be a positive pressure value, that is, the target pressure value $P_t$ is larger than the atmospheric pressure value. Because in the embodiment of the present disclosure, the ventilation therapy apparatus uses an open gas path, it is necessary to ensure that the positive pressure is maintained in the nasal cavity, to ensure that the human body will not directly inhale outside air.

Step 503, calculating an actual pressure value at the patient interface according to the output pressure value and the output flow value of the signal collection point.

In this step, either the theoretical output flow rate $F_t$ may be calculated from $P_1$, or the pressure drop $\Delta P$ of the air flow through the tube may be calculated from $F_0$, and according to the formula $P_1 = \Delta P + P_2$, the value of the airflow pressure $P_2$ at the patient interface may be obtained.

Optionally, the step 503 further comprises:

Sub-step 5031, acquiring a gas resistance pressure value from the signal collection point to the patient interface.

For details of this step, reference may be made to the above description of the air resistance pressure acquisition module 1031, which is not repeated here.

Sub-step 5032, subtracting the gas resistance pressure value from the output pressure value, and obtaining the actual pressure value.

For details of this step, reference may be made to the above description of the second calculation module 1032, which is not repeated here.

Optionally, the step 503 further comprises:

Sub-step 5033, acquiring the output pressure value and the output flow value of the signal collection point of the apparatus body;

For details of this step, reference may be made to the above description of the flow acquisition module 10311, which is not repeated here.

Sub-step 5034, acquiring, under different pressure states, corresponding test flow values when the patient interface is vacant, and acquiring a gas resistance characteristic from the signal collection point to the patient interface, the gas resistance characteristic includes a correspondence relationship between the gas resistance pressure value and the output flow value.

For details of this step, reference may be made to the above description of the gas resistance characteristic acquisition module 10312, which is not repeated here.

Sub-step 5035, according to the output flow value of the apparatus body in working state and the corresponding gas resistance characteristic, acquiring the corresponding gas resistance pressure value.

For details of this step, reference may be made to the above description of the gas resistance pressure acquisition unit 10313, which is not repeated here.

Sub-step 5036, subtracting the corresponding gas resistance pressure value from the output pressure value of the apparatus body, and obtaining the actual pressure value.

For details of this step, reference may be made to the above description of the second calculation module 1032, which is not repeated here.

Step 504, adjusting an output flow of the apparatus body according to the actual pressure value and the target pressure value.

Step 505, when the actual pressure value is larger than the target pressure value, reducing the output flow of the apparatus body.

In the example of the present disclosure, before the patient uses the air supply system of the ventilation therapy apparatus, the target pressure value $P_t$ will be preset according to their own conditions, and the target pressure value $P_t$ is the pressure value in an ideal state free from the interference of the pressure drop. When the patient uses the ventilation therapy apparatus, due to the interference of the pressure drop, which will cause the actual pressure $P_2$ at the patient interface is different from the target pressure value $P_t$, and the influence of this pressure drop on the actual pressure $P_2$ at the patient interface may be determined by the first control module based on the comparison result between $P_2$ and $P_t$. If the first control module determines that the actual pressure $P_2$ at the patient interface is larger than the target pressure value $P_t$, it is determined that the current working state of the apparatus body 10 is the exhalation state, meanwhile a control instruction is sent to the motor by the first control module, therefore the motor drives the fan to work at a lower rotating speed, and output a smaller first output threshold airflow, to prevent the patient's exhaled gas from flowing back to the ventilation therapy apparatus, until the actual pressure value is equal to the target pressure value.

Step 506, when the actual pressure value is less than the target pressure value, rising the output flow of the apparatus body.

In this step, if the first control module determines that the actual pressure $P_2$ at the patient interface is less than the target pressure value $P_t$, then it is determined that the current working state of the apparatus body is the inhalation state. At this time, a control instruction is sent to the motor by the first control module, therefore the motor drives the fan to work at a relatively high rotating speed, and output a larger second output threshold airflow, to perform auxiliary inhalation.

Optionally, in an implementation manner, it may also include:

Step A1, when the patient interface is worn on the patient's nasal cavity, adjusting the output pressure value of the apparatus body to the target pressure value; and

Step A2, when the patient interface is not worn on the patient's nasal cavity, adjusting the output pressure value of the apparatus body to a preset pressure value which is less than the target pressure value, or controlling the apparatus body to stop running.

In the example of the present disclosure, when the pressure $P_2$ at the patient interface is close to 0, it is in the standby state, when the airflow pressure at the patient interface is close to 0 and maintained for the preset time, it means that the patient interface is not worn on the patient's nasal cavity, the patient does not use the air supply system of the ventilation therapy apparatus, and it is the standby state. When the patient interface is exposed to the air, at this time, the ventilation therapy apparatus may continue to output smaller output flow, to ensure the temperature and the humidity inside the respiratory pipe are constant, or directly control the apparatus body to stop running, to save power.

In addition, when the patient interface is worn on the patient's nasal cavity, the second control module is configured for automatically adjusting the output pressure value of the apparatus body to the target pressure value, therefore the patient may receive oxygen supply quickly.

Optionally, in another implementation manner, it may also include:

Step B1, determining a respiratory phase according to the output pressure value and the output flow value, the respiratory phase includes the inspiratory phase and the exhalation phase.
Step B2, acquiring a target pressure value of the inspiratory phase and a target pressure value of the exhalation phase at the patient interface.

Specifically, the patient may preset the target pressure value of the inspiration phase and the target pressure value of the expiration phase, and the target pressure value of the inspiration phase and the target pressure value of the expiration phase are the pressures of the patient interface during inhalation and exhalation in an ideal state.

Step B3, if it is determined that the he current is inspiratory phase, adjusting the output flow of the apparatus body according to the actual pressure value and the target pressure value of the inspiratory phase.

When it is determined that the patient is inhaling, the ventilation therapy apparatus, under the control of the first control module, makes the motor drive the fan to work at a relatively large rotating speed, therefore the ventilation therapy apparatus outputs a flow that is slightly larger than the patient's inhalation volume, until the pressure value at the patient interface reaches the target pressure value of the inhalation phase, to perform auxiliary inhalation.

Step B4, if it is determined that the he current is exhalation phase, adjusting the output flow of the apparatus body according to the actual pressure value and the target pressure value of the exhalation phase.

When it is determined that the patient is exhaling, the ventilation therapy apparatus, under the control of the first control module, makes the motor drive the fan to work at a lower rotating speed, therefore the ventilation therapy apparatus outputs a smaller flow rate, until the pressure value at the patient interface reaches the target pressure value of the exhalation stage, to prevent the patient's exhaled gas from flowing back to the ventilation therapy apparatus.

In summary, the method for controlling the ventilation therapy apparatus according to the example of the present disclosure, includes: acquiring an output pressure value and an output flow value of a signal collection point of an apparatus body; acquiring a target pressure value at a patient interface; calculating an actual pressure value at a patient interface according to the output pressure value and the output flow value of the signal collection point; adjusting the output flow of the apparatus body according to the actual pressure value and the target pressure value; when the actual pressure value is larger than the target pressure value, reducing the output flow of the apparatus body; and when the actual pressure value is less than the target pressure value, rising the output flow of the apparatus body. In the present disclosure, it is capable to determine the actual pressure value at the patient interface by the output parameters feedback of the signal collection point of the apparatus body, determine the patient's respiratory state according to the comparison between the actual pressure value and the target pressure value, and output the gas with a corresponding threshold, therefore the gas pressure of the airflow received by the patient may reach a preset target pressure range, and achieve the therapeutic effect.

The various component examples of the present disclosure may be implemented by hardware, or by software modules running on one or more processors, or by a combination of them. Those skilled in the art should understand that, a microprocessor or a digital signal processor (DSP) may be used in practice to implement some or all of the functions of some or all of the components in the computing processing device according to the embodiments of the present disclosure. The present disclosure may also be implemented as a device or device program (for example, a computer program and a computer program product) for executing part or all of the methods described herein. Such a program for implementing the present application may be stored on a computer-readable medium, or may have the form of one or more signals. Such a signal may be downloaded from an Internet website, or provided on a carrier signal, or provided in any other form.

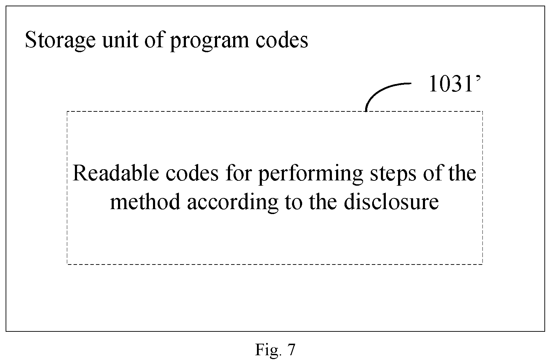

For example, Fig. 6 is a computing processing device that may implement the method according to the present disclosure provided in an example of the present disclosure. The computing processing device traditionally includes a processor 1010 and a computer program product in the form of a memory 1020 or a computer readable medium. The memory 1020 may be an electronic memory such as flash memory, EEPROM (Electrically Erasable Programmable Read Only Memory), EPROM, hard disk, or ROM. The memory 1020 has a storage space 1030 for executing the program code 1031 of any method step in the above method. For example, the storage space 1030 for program codes may include various program codes 1031 respectively used to implement various steps in the above method. These program codes may be read from or written into one or more computer program products. These computer program products include program code carriers such as hard disks, compact disks (CDs), memory cards, or floppy disks. Such computer program products are usually portable or fixed storage modules as described with reference to Fig. 7. The storage module may have storage segments, storage spaces, etc., arranged similarly to the memory 1020 in the computing processing device of Fig. 6. The program code may be compressed in an appropriate form, for example. Generally, the storage module includes computer-readable code 1031', that is, code that may be read by a processor such as 1010, which, when run by the computing processing device, causes the computing processing device to execute the various steps of the method described above.

In this application, a computer-readable recording medium includes any mechanism for storing or transmitting information in a computer (for example, a computer) readable form. For example, machine-readable medias include read-only memory (ROM), random access memory (RAM), magnetic disk storage media, optical storage media, flash storage media, electrical, optical, acoustic, or other forms of propagated signals (for example, carrier waves, infrared signal, digital signal, etc.) etc.

The invention is defined by the appended claims. Subject-matters referred as embodiments and/or disclosures which are not claimed are not part of the invention.

**Claims**

1. A ventilation therapy apparatus, comprising:

    an apparatus body (10), configured for outputting gas with a preset pressure and a preset flow, wherein the apparatus body (10) includes a gas outlet;
    a respiratory pipe (20), including a first end (201) and a second end (202) which communicates with each other, wherein the first end (201) of the respiratory pipe (20) communicates with the gas outlet;
    a patient interface (30), wherein the second end (202) of the respiratory pipe (20) is connected to the patient interface (30), the patient interface (30) is configured for being worn on a patient's nasal cavity, when the patient interface (30) is worn on the patient's nasal cavity, a gas outlet gap is disposed between the patient interface (30) and the patient's nasal cavity;
    wherein the apparatus body (10) further includes:

        a signal acquisition module (101), configured for acquiring an output pressure value and an output flow value of a signal collection point of the apparatus body (10);
        a target pressure acquisition module (102), configured for acquiring a target pressure value at the patient interface (30);
        a first calculation module (103) configured for, calculating an actual pressure value at the patient interface (30) according to the output pressure value and the output flow value of the signal collection point;
        a first control module (104) configured for, adjusting an output flow of the apparatus body (10) according to the actual pressure value and the target pressure value;
        when the actual pressure value is larger than the target pressure value, reducing the output flow of the apparatus body (10);
        when the actual pressure value is less than the target pressure value, rising the output flow of the apparatus body (10);
        wherein the first calculation module (103) comprises:

            a gas resistance pressure acquisition module (1031), configured for acquiring a gas resistance pressure value from the signal collection point to the patient interface (30); and
            a second calculation module (1032), configured for subtracting the gas resistance pressure value from the output pressure value, and obtaining the actual pressure value;
            wherein the gas resistance pressure acquisition module (1031) comprises:

                a gas resistance characteristic acquisition module (10312), configured for, acquiring, under different pressure states, corresponding test flow values through a flow acquisition module (10311) when the patient interface (30) is vacant, and acquiring a gas resistance characteristic from the signal collection point to the patient interface (30), wherein the gas resistance characteristic includes a correspondence relationship between the gas resistance pressure value and the output flow value;
                a gas resistance pressure acquisition unit (10313), configured for, acquiring the corresponding gas resistance pressure value according to the output flow value of the apparatus body (10) in working state and the corresponding gas resistance characteristic; wherein the second calculation module (1032) is further configured for, subtracting the corresponding gas resistance pressure value from the output pressure value of the apparatus body (10), and obtaining the actual pressure value.

2. The ventilation therapy apparatus according to claim 1, wherein the signal collection point is disposed at the gas outlet of the apparatus body (10).

3. The ventilation therapy apparatus according to claims 1 or 2, wherein the target pressure value comprises a target pressure value of an inspiratory phase and a target pressure value of an exhalation phase;

the ventilation therapy apparatus includes a determination module, the determination module is configured for determining a respiratory phase according to the output pressure value and the output flow value acquired by the signal acquisition module (101), the respiratory phase including the inspiratory phase and the exhalation phase;

when the determination module determines that the current is the inspiratory phase, the first control module (104) adjusts the output flow of the apparatus body (10) according to the actual pressure value and the target pressure value of the inspiratory phase; and when the determination module determines that the current is the exhalation phase, the first control module (104) adjusts the output flow of the apparatus body (10) according to the actual pressure value and the target pressure value of the exhalation phase.

4. The ventilation therapy apparatus according to any one of claims 1 to 3, wherein the apparatus body (10) further comprises a positive pressure gas source and a humidifier, the positive pressure gas source is configured for providing an output gas, and the humidifier is configured for heating and humidifying the output gas, wherein the humidifier is connected to an output end of the positive pressure gas source.

5. The ventilation therapy apparatus according to claim 4, wherein the positive pressure gas source comprises a gas source body capable of outputting gas with a preset flow, and/or a centrifugal fan configured for pressurize air, wherein the maximum rotation speed of the centrifugal fan is larger than or equal to 20000r/min.

6. The ventilation therapy apparatus according to any one of claims 1 to 5, wherein the respiratory pipe (20) further comprises a heating element configured for heating gas passing through the respiratory pipe (20), the rated power of the heating element is larger than 20 watts.

7. The ventilation therapy apparatus according to claim 6, wherein the respiratory pipe (20) further comprises a temperature sensor, configured for monitoring the temperature of the gas passing through the respiratory pipe (20).

8. The ventilation therapy apparatus according to any one of claims 1 to 7, wherein the respiratory pipe (20) and the apparatus body (10) are connected through a gas path and a circuit, and the circuit and the gas path are on and off simultaneously.

9. The ventilation therapy apparatus according to any one of claims 1 to 8, wherein the ventilation therapy apparatus comprises a second control module; the second control module being configured such that when the patient interface (30) is worn on the patient's nasal cavity, the second control module is configured for adjusting the output pressure value of the apparatus body (10) to the target pressure value; and
when the patient interface (30) is not worn on the patient's nasal cavity, the second control module is configured for adjusting the output pressure value of the apparatus body (10) to a preset pressure value, or the second control module is configured for controlling the apparatus body (10) to stop running.

**Patentansprüche**

1. Atemtherapievorrichtung, die umfasst:

einen Vorrichtungskörper (10), der zum Ausgeben von Gas mit einem voreingestellten Druck und einem voreingestellten Fluss konfiguriert ist, wobei der Vorrichtungskörper (10) einen Gasauslass aufweist;
ein Beatmungsrohr (20), das ein erstes Ende (201) und ein zweites Ende (202) aufweist, die miteinander kommunizieren, wobei das erste Ende (201) des Beatmungsrohrs (20) mit dem Gasauslass kommuniziert;
eine Patientenschnittstelle (30), wobei das zweite Ende (202) des Beatmungsrohrs (20) mit der Patientenschnittstelle (30) verbunden ist, wobei die Patientenschnittstelle (30) so konfiguriert ist, dass sie an der Nasenöffnung eines Patienten getragen wird, wobei, wenn die Patientenschnittstelle (30) an der Nasenöffnung des Patienten getragen wird, ein Gasauslassspalt zwischen der Patientenschnittstelle (30) und der Nasenöffnung des Patienten angeordnet ist;

wobei der Vorrichtungskörper (10) des Weiteren aufweist:

ein Signalerfassungsmodul (101), das zum Erfassen eines Ausgangsdruckwertes und eines Ausgangsflusswertes eines Signalsammelpunktes des Vorrichtungskörpers (10) konfiguriert ist;

ein Soll-Druckerfassungsmodul (102), das zum Erfassen eines Solldruckwertes an der Patientenschnittstelle (30) konfiguriert ist;

ein erstes Berechnungsmodul (103) das zum Berechnen eines Ist-Druckwertes an der Patientenschnittstelle (30) entsprechend dem Ausgangsdruckwert und dem Ausgangsflusswert des Signalsammelpunktes konfiguriert ist;

ein erstes Steuerungsmodul (104), das zum Anpassen eines Ausgangsflusses des Vorrichtungskörpers (10) entsprechend dem Ist-Druckwert und dem Soll-Druckwert konfiguriert ist;

wenn der Ist-Druckwert größer ist als der Soll-Druckwert, Verringern der Ausgangsflusses des Vorrichtungskörpers (10);

wenn der Ist-Druckwert kleiner ist als der Soll-Druckwert, Erhöhen des Ausgangsflusses des Vorrichtungskörpers (10);

wobei das erste Berechnungsmodul (103) umfasst:

ein Gaswiderstandsdruck-Erfassungsmodul (1031), das zum Erfassen eines Gaswiderstandsdruckwertes vom Signalsammelpunkt zur Patientenschnittstelle (30) konfiguriert ist; und

ein zweites Berechnungsmodul (1032), das zum Abziehen des Gaswiderstandsdruckwertes vom Ausgangsdruckwert und Beziehen des Ist-Druckwertes konfiguriert ist;

wobei das Gaswiderstandsdruck-Erfassungsmodul (1031) umfasst:

ein Gaswiderstandscharakteristik-Erfassungsmodul (10312), das zum Erfassen unter unterschiedlichen Druckzuständen entsprechender Testflusswerte durch ein Flusserfassungsmodul (10311), wenn die Patientenschnittstelle (30) frei ist, und Erfassen einer Gaswiderstandscharakteristik von dem Signalsammelpunkt zu der Patientenschnittstelle (30) konfiguriert ist, wobei die Gaswiderstandscharakteristik eine Korrespondenzbeziehung zwischen dem Gaswiderstandsdruckwert und dem Ausgangsflusswert aufweist;

eine Gaswiderstandsdruck-Erfassungseinheit (10313), die zum Erfassen des entsprechenden Gaswiderstandsdruckwertes entsprechend dem Ausgangsflusswert des Vorrichtungskörpers (10) im Arbeitszustand und der entsprechenden Gaswiderstandscharakteristik konfiguriert ist; wobei das zweite Berechnungsmodul (1032) des Weiteren zum Abziehen des entsprechenden Gaswiderstandsdruckwertes von dem Ausgangsdruckwert des Vorrichtungskörpers (10) und Beziehen des tatsächlichen Druckwertes konfiguriert ist.

2. Atemtherapievorrichtung nach Anspruch 1, wobei der Signalsammelpunkt an dem Gasauslass des Vorrichtungskörpers (10) angeordnet ist.

3. Atemtherapievorrichtung nach Anspruch 1 oder 2, wobei der Soll-Druckwert einen Soll-Druckwert einer Einatmungsphase und einen Solldruckwert einer Ausatmungsphase umfasst;

die Atemtherapievorrichtung ein Bestimmungsmodul aufweist, wobei das Bestimmungsmodul zum Bestimmen einer Atmungsphase entsprechend dem von dem Signalerfassungsmodul (101) erfassten Ausgangsdruckwert und Ausgangsflusswert konfiguriert ist, wobei die Atmungsphase die Einatmungsphase und die Ausatmungsphase aufweist;

wenn das Bestimmungsmodul bestimmt, dass die aktuelle die Einatmungsphase ist, das erste Steuerungsmodul (104) den Ausgangsfluss des Vorrichtungskörpers (10) entsprechend dem Ist-Druckwert und dem Soll-Druckwert der Einatmungsphase anpasst; und

wenn das Bestimmungsmodul bestimmt, dass die aktuelle die Ausatmungsphase ist, das erste Steuerungsmodul (104) den Ausgangsfluss des Vorrichtungskörpers (10) entsprechend dem Ist-Druckwert und dem Soll-Druckwert der Ausatmungsphase anpasst.

4. Atemtherapievorrichtung nach einem der Ansprüche 1 bis 3, wobei der Vorrichtungskörper (10) des Weiteren eine Überdruckgasquelle und einen Befeuchter umfasst, wobei die Überdruckgasquelle zum Bereitstellen eines Ausgangsgases konfiguriert ist und der Befeuchter zum Erwärmen und Befeuchten des Ausgangsgases konfiguriert ist, wobei der Befeuchter mit einem Ausgangsende der Überdruckgasquelle verbunden ist.

5. Atemtherapievorrichtung nach Anspruch 4, wobei die Überdruckgasquelle einen Gasquellenkörper, der Gas mit einem voreingestellten Fluss ausgeben kann, und/oder ein Zentrifugalgebläse umfasst, das zum Beaufschlagen der Luft mit Druck konfiguriert ist, wobei die maximale Drehzahl des Zentrifugalgebläses größer oder gleich 20000 U/min ist.

6. Atemtherapievorrichtung nach einem der Ansprüche 1 bis 5, wobei das Beatmungsrohr (20) des Weiteren ein Heizelement umfasst, das zum Erwärmen von durch das Beatmungsrohr (20) strömendem Gas konfiguriert ist, wobei die Nennleistung des Heizelements größer ist als 20 Watt.

7. Atemtherapievorrichtung nach Anspruch 6, wobei das Beatmungsrohr (20) des Weiteren einen Temperatursensor umfasst, der zur Überwachung der Temperatur des durch das Beatmungsrohr (20) strömenden Gases konfiguriert ist.

8. Atemtherapievorrichtung nach einem der Ansprüche 1 bis 7, wobei das Beatmungsrohr (20) und der Vorrichtungskörper (10) durch einen Gasweg und einen Kreislauf verbunden sind, wobei der Kreislauf und der Gasweg gleichzeitig ein- und ausgeschaltet sind.

9. Atemtherapievorrichtung nach einem der Ansprüche 1 bis 8, wobei die Atemtherapievorrichtung ein zweites Steuerungsmodul umfasst; wobei das zweite Steuerungsmodul so konfiguriert ist, dass, wenn die Patientenschnittstelle (30) an der Nasenöffnung des Patienten getragen wird, das zweite Steuerungsmodul zum Anpassen des Ausgangsdruckwertes des Vorrichtungskörpers (10) auf den Soll-Druckwert konfiguriert ist; und
wenn die Patientenschnittstelle (30) nicht an der Nasenöffnung des Patienten getragen wird, das zweite Steuerungsmodul zum Anpassen des Ausgangsdruckwertes des Vorrichtungskörpers (10) auf einen voreingestellten Druckwert konfiguriert ist, oder das zweite Steuerungsmodul zum Steuern des Vorrichtungskörpers (10) so konfiguriert ist, dass er nicht mehr ausgeführt wird.

**Revendications**

1. Appareil de thérapie par ventilation, comprenant :

un corps d'appareil (10), configuré pour délivrer en sortie du gaz avec une pression prédéfinie et un débit prédéfini, dans lequel le corps d'appareil (10) comprend une sortie de gaz ;
un tuyau respiratoire (20), comprenant une première extrémité (201) et une deuxième extrémité (202) qui communiquent l'une avec l'autre, où la première extrémité (201) du tuyau respiratoire (20) communique avec la sortie de gaz ;
une interface patient (30), où la deuxième extrémité (202) du tuyau respiratoire (20) est reliée à l'interface patient (30), l'interface patient (30) est configurée pour être portée sur la cavité nasale d'un patient, lorsque l'interface patient (30) est portée sur la cavité nasale du patient, un espace de sortie de gaz est disposé entre l'interface patient (30) et la cavité nasale du patient ;
dans lequel le corps d'appareil (10) comprend en outre :

un module d'acquisition de signal (101), configuré pour acquérir une valeur de pression de sortie et une valeur de débit de sortie d'un point de collecte de signal du corps d'appareil (10) ;
un module d'acquisition de pression cible (102), configuré pour acquérir une valeur de pression cible au niveau de l'interface patient (30) ;
un premier module de calcul (103) configuré pour calculer une valeur de pression réelle au niveau de l'interface patient (30) selon la valeur de pression de sortie et la valeur de débit de sortie du point de collecte de signal ;
un premier module de commande (104) configuré pour ajuster un débit de sortie du corps d'appareil (10) selon la valeur de pression réelle et la valeur de pression cible ;
lorsque la valeur de pression réelle est supérieure à la valeur de pression cible, réduire le débit de sortie du corps d'appareil (10) ;
lorsque la valeur de pression réelle est inférieure à la valeur de pression cible, augmenter le débit de sortie du corps d'appareil (10) ;

dans lequel le premier module de calcul (103) comprend :

un module d'acquisition de pression de résistance de gaz (1031), configuré pour acquérir une valeur de pression de résistance de gaz du point de collecte de signal à l'interface patient (30) ; et
un deuxième module de calcul (1032), configuré pour soustraire la valeur de pression de résistance de gaz de la valeur de pression de sortie, et obtenir la valeur de pression réelle ;
dans lequel le module d'acquisition de pression de résistance de gaz (1031) comprend :

un module d'acquisition de caractéristique de résistance de gaz (10312), configuré pour acquérir, sous différents états de pression, des valeurs de débit de test correspondantes par un module d'acquisition de débit (10311) lorsque l'interface patient (30) est vide, et acquérir une caractéristique de résistance de gaz du point de collecte de signal à l'interface patient (30), où la caractéristique de résistance de gaz comprend une relation de correspondance entre la valeur de pression de résistance de gaz et la valeur de débit de sortie ;
une unité d'acquisition de pression de résistance de gaz (10313), configurée pour acquérir la valeur de pression de résistance de gaz correspondante selon la valeur de débit de sortie du corps d'appareil (10) en état de fonctionnement et la caractéristique de résistance de gaz correspondante ;
dans lequel le deuxième module de calcul (1032) est en outre configuré pour soustraire la valeur de pression de résistance de gaz correspondante de la valeur de pression de sortie du corps d'appareil (10) et obtenir la valeur de pression réelle.

2. Appareil de thérapie par ventilation selon la revendication 1, dans lequel le point de collecte de signal est disposé au niveau de la sortie de gaz du corps d'appareil (10).

3. Appareil de thérapie par ventilation selon la revendication 1 ou 2, dans lequel la valeur de pression cible comprend une valeur de pression cible d'une phase d'inspiration et une valeur de pression cible d'une phase d'exhalation ;

l'appareil de thérapie par ventilation comprend un module de détermination, le module de détermination est configuré pour déterminer une phase respiratoire selon la valeur de pression de sortie et la valeur de débit de sortie acquises par le module d'acquisition de signal (101), la phase respiratoire comprenant la phase d'inspiration et la phase d'exhalation ;
lorsque le module de détermination détermine que la phase actuelle est la phase d'inspiration, le premier module de commande (104) ajuste le débit de sortie du corps d'appareil (10) selon la valeur de pression réelle et la valeur de pression cible de la phase d'inspiration ; et
lorsque le module de détermination détermine que la phase actuelle est la phase d'exhalation, le premier module de commande (104) ajuste le débit de sortie du corps d'appareil (10) selon la valeur de pression réelle et la valeur de pression cible de la phase d'exhalation.

4. Appareil de thérapie par ventilation selon l'une quelconque des revendications 1 à 3, dans lequel le corps d'appareil (10) comprend en outre une source de gaz à pression positive et un humidificateur, la source de gaz à pression positive est configurée pour fournir un gaz de sortie, et l'humidificateur est configuré pour chauffer et humidifier le gaz de sortie, où l'humidificateur est relié à une extrémité de sortie de la source de gaz à pression positive.

5. Appareil de thérapie par ventilation selon la revendication 4, dans lequel la source de gaz à pression positive comprend un corps de source de gaz capable de délivrer en sortie du gaz avec un débit prédéfini, et/ou un ventilateur centrifuge configuré pour pressuriser l'air, où la vitesse de rotation maximale du ventilateur centrifuge est supérieure ou égale à 20000 tr/min.

6. Appareil de thérapie par ventilation selon l'une quelconque des revendications 1 à 5, dans lequel le tuyau respiratoire (20) comprend en outre un élément chauffant configuré pour chauffer le gaz traversant le tuyau respiratoire (20), la puissance nominale de l'élément chauffant est supérieure à 20 watts.

7. Appareil de thérapie par ventilation selon la revendication 6, dans lequel le tuyau respiratoire (20) comprend en outre un capteur de température, configuré pour surveiller la température du gaz traversant le tuyau respiratoire (20).

8. Appareil de thérapie par ventilation selon l'une quelconque des revendications 1 à 7, dans lequel le tuyau respiratoire (20) et le corps d'appareil (10) sont reliés par un trajet de gaz et un circuit, et le circuit et le trajet de gaz sont activés et désactivés simultanément.

9. Appareil de thérapie par ventilation selon l'une quelconque des revendications 1 à 8, dans lequel l'appareil de thérapie par ventilation comprend un deuxième module de commande ; le deuxième module de commande étant configuré de sorte que, lorsque l'interface patient (30) est portée sur la cavité nasale du patient, le deuxième module de commande soit configuré pour ajuster la valeur de pression de sortie du corps d'appareil (10) à la valeur de pression cible ; et

lorsque l'interface patient (30) n'est pas portée sur la cavité nasale du patient, le deuxième module de commande est configuré pour ajuster la valeur de pression de sortie du corps d'appareil (10) à une valeur de pression prédéfinie, ou le deuxième module de commande est configuré pour commander le corps d'appareil (10) pour arrêter de fonctionner.

10

201

20

202

30

Apparatus body

| Signal acquisition module | 101 |

| Target pressure acquisition module | 102 |

103

| First calculation module | |

104

| First control module | |

105

| Fan | |

Fig. 1

Flow

——— Patient respiratory flow curve

– – – Ventilator output flow curve

–·–·– Ventilator output Oxygen flow curve

Patient's inhaling

Time

Patient's exhaling

Fig.2

Fig. 3

Fig.4

acquiring an output pressure value and an output flow value of a signal collection point of an apparatus body — 501

acquiring a target pressure value at a patient interface — 502

calculating an actual pressure value at the patient interface according to the output pressure value and the output flow value of the signal collection point — 503

adjusting an output flow of the apparatus body according to the actual pressure value and the target pressure value — 504

when the actual pressure value is greater than the target pressure value, reducing the output flow of the apparatus body — 505

when the actual pressure value is less than the target pressure value, rising the output flow of the apparatus body — 506

Fig. 5

Processor 1010

Memory 1020

Space of program codes 1030

Program codes for performing steps of the method according to the disclosure

Computing processing device

Fig. 6

Storage unit of program codes

1031'

Readable codes for performing steps of the
method according to the disclosure

Fig. 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 201811261603
- WO 2009146484 A1
- WO 93709834 A1
- WO 02720076 A1
- WO 2011141845 A1